# EUROPEAN PATENT APPLICATION

(11) **EP 3 888 633 A1**
(43) Date of publication of application: **06.10.2021**
(21) Application number: 19891569.6
(22) Date of filing: 29.11.2019
(51) Int. Cl.: A61K 9/08, A61K 47/26, A61K 47/18, A61K 47/20, A61K 47/64, A61K 38/48

(54) **BOTULINUM TOXIN-STABILIZING LIQUID COMPOSITION**

(30) Priority: 30.11.2018 KR 20180151983; 20.08.2019 KR 20190101723
(71) Applicant: Huons Biopharma Co., Ltd., Seongnam-si, Gyeonggi-do 13201 (KR)
(72) Inventor: KIM, Wanseop Paul, Yongin-si, Gyeonggi-do 16822 (KR); KIM, Yeong-Mok, Seoul 04732 (KR); PARK, Gi-Sik, Suwon-si, Gyeonggi-do 16697 (KR); PARK, Jae-Min, Suwon-si, Gyeonggi-do 16281 (KR); LEE, Ae-Yeon, Ansan-si, Gyeonggi-do 15589 (KR)
(74) Representative: Dehns
(86) International application number: PCT/KR2019/016669
(87) International publication number: WO 2020/111852

(57) **Abstract**

The present invention relates to a botulinum toxin-stabilizing liquid composition having the effect of improving the stability of botulinum toxin, a method for preparing same, and a pharmaceutical composition containing the stabilizing liquid composition and having improved stability of botulinum toxin. According to the present invention, since the activity of liquid botulinum toxin is maintained for a long time even at room temperature, there are advantages of excellent storage stability and conservation stability and easy administration.

## Description

### [Technical Field]

The present invention relates to a stabilizing liquid composition having an effect of improving the stability of botulinum toxin, a method of preparing the same, and a pharmaceutical composition for improving the stability of botulinum toxin, which includes the stabilizing liquid composition.

The application claims priority to and the benefit of Korean Patent Application No. 10-2018-0151983, filed on November 30, 2018, and Korean Patent Application No. 10-2019-0101723, filed on August 20, 2019, the disclosures of which are incorporated herein by reference in their entireties.

### [Background Art]

There are 127 species or more classified by morphology and function in the genus Clostridium. Anaerobic, gram-positive bacteria, *Clostridium botulinum*, generates a potent polypeptide neurotoxin, such as botulinum toxin (hereinafter, the "toxin" used herein is also used interchangeably), causing a neuroparalytic disease known as botulism in humans and animals. Symptoms of the botulinum toxin poisoning may include gait abnormalities, dysphagia, speech disorders, and paralysis of respiratory muscles which may lead to death.

One unit of the botulinum toxin is defined as LD₅₀ when intraperitoneally injected into female Swiss Webster mice weighing approximately 18 to 20 g. One unit of the botulinum toxin is the amount of botulinum toxin that kills 50% of a group of female Swiss Webster mouse.

The characteristics of seven types of immunologically distinct botulinum neurotoxins, such as botulinum neurotoxin serotypes A, B, C, D, E, F and G, which are distinguished by being neutralized with a type-specific antibody. According to the different serotypes of botulinum toxin, there are differences in the animal species affected thereby, and the severity and duration of paralysis caused thereby. Botulinum toxin apparently binds to cholinergic motor neurons with high affinity, relocates to a neuron, and blocks the presynaptic release of acetylcholine.

Botulinum toxin has been used in a clinical setting for treating neuromuscular disorders characterized by hyperactive skeletal muscle. Botulinum toxin type A is approved by the U.S. Food and Drug Administration (FDA) for essential blepharopasm, strabismus and hemifacial spasm in patients over age 12, and also approved for the treatment of cervical dystonia, glabellar (facial) wrinkles and hyperhidrosis. The FDA also approved botulinum toxin type B for the treatment of cervical dystonia, glabellar (facial) wrinkles.

Botulinum toxin type A is known to be soluble in a dilute aqueous solution with pH 4 to 6.8. A stabilizing non-toxic protein is separated from a neurotoxin at pH of approximately 7 or more, resulting in a gradual loss of toxicity, especially as the pH and temperature rise. As is common with enzymes, the biological activity of botulinum toxin (which is an intracellular peptidase) is at least partially dependent on the three-dimensional morphology thereof. Dilution to a solution containing of milligrams to nanograms of toxin per mL has considerable difficulty, for example, the tendency of reducing an amount of available toxin by the toxin adhering to a surface. In addition, a toxin-containing pharmaceutical composition is sometimes used months or years after preparation, and in this case, the toxin may be easily oxidized or decomposed into small fragments due to the nature of a protein. Therefore, it is necessary to stabilize the toxin with a stabilizer.

Conventionally, to solve this problem, albumin, sucrose, trehalose and/or gelatin are mainly used as a stabilizer in the pharmaceutical process of a botulinum toxin protein.

For example, as described below, for a botulinum toxin-containing pharmaceutical composition, albumin is used as a stabilizer.

A commercially available botulinum toxin-containing pharmaceutical composition is commercialized by Allergan, Inc (Irvine, CA) under the trade name BOTOX^{®} (botulinum toxin type A, purified neurotoxin complex). 100 units/vial of BOTOX^{®} consists of approximately 5 ng of the purified botulinum toxin type A complex, 0.5 mg human serum albumin, and 0.9 mg sodium chloride, and is in a vacuum-dried form, and prepared to be restored with sterile physiological saline without a preservative (injected with 0.9% sodium chloride). Other commercially available botulinum toxin-containing pharmaceutical compositions may include Dysport^{®} *(Clostridium botulinum* type A toxin-hemagglutinin complex having lactose and human serum albumin among a botulinum toxin pharmaceutical composition, commercially available from Ipsen Limited, Berkshire, UK, a powder to be restored with 0.9% sodium chloride before use), and MyoBloc™ (an injectable solution containing botulinum toxin type B, human serum albumin, sodium succinate and sodium chloride at approximately pH 5.6, commercially available from Solstice Neurosciences, San Diego, CA). A neurotoxin component (150 kDa toxin molecule) and a botulinum toxin complex (300 kDa to 900 kDa) may be obtained from, for example, List Biological Laboratories, Inc (Campbell, CA); the Centre for Applied Microbiology and Research (Porton Down, UK); and Wako (Osaka, Japan), and also from Sigma Chemicals (Saint Louis, MI).

As described above, the reason why albumin is mainly used is that albumin may not only stabilize a protein active ingredient in a pharmaceutical composition, but may also have the advantage of negligible immunogenicity when injected into human patients. However, blood products such as albumin may pose a potential risk of infecting a patient with a blood-derived pathogen or infectious microorganism, and particularly, the mediation of diseases such as Creutzfeldt-Jacob disease delivered by viral infection or a viral protein may not be completely eliminated, and there may also be a risk of infecting a patient with a so far unknown pathogen. Therefore, when the above-described albumin is used as a stabilizer, selection of a donor or donation site is performed in a preparation process and the removal and/or inactivation of an infectious material should be performed to reduce the risk of spread of the infectious material.

In addition, gelatin is sometimes used. However, since it is also a protein obtained from an animal, it may cause a disease like albumin, and therefore may not be recommended for use in a pharmaceutical process.

As technology for using a stabilizer which is not derived from an animal source in a pharmaceutical process for a botulinum toxin protein, a method of utilizing a pharmaceutical preparation of recombinant albumin (rSA) produced in yeasts is disclosed in Korean Registered Patent No. 10-0799400. However, since a novel antigenic structure, neoepitope, is generated in the production and isolation and recovery of recombinant albumin (rSA), the possibility of inducing an immune response in a recipient of the drug may not be completely eliminated.

In addition, in Korean Registered Patent No. 10-0665469, a pharmaceutical composition containing botulinum toxin, a polysaccharide (including hydroxyethyl starch), and lysine, glycine, histidine or arginine as an amino acid is disclosed. However, the pharmaceutical composition of the present invention is a composition for a freeze-dried formulation, and its storage condition is limited to freezing or refrigeration. The freeze-dried botulinum toxin needs a process of melting or diluting a frozen formulation immediately before use, and in this process, an error may be generated. Moreover, the freeze-dried botulinum toxin is inconvenient in use, and has a problem of impossible development in a prefilled syringe type.

Regarding a composition for stabilizing botulinum toxin, in U.S. Unexamined Patent Application Publication No. 2007-0134199, a composition containing a non-ionic surfactant containing polysorbate, and glutamine and glutamic acid or asparagine and aspartic acid as amino acids is disclosed. When botulinum toxin is diluted in the liquid composition of the present invention and stored under a refrigeration condition of 4 °C, it is stabilized over 8 months, but when the botulinum toxin is stored at room temperature, for example, 37 °C, its activity is reduced within one month, so that there is a limit to stabilization of a pharmaceutical liquid composition containing botulinum toxin at room temperature.

### [Disclosure]

### [Technical Problem]

The present invention is directed to providing a stabilizing liquid composition which is safe for humans since it is not derived from an animal source, and improves the stability of a botulinum toxin solution to maintain the activity of botulinum toxin at room temperature, and a botulinum toxin solution with improved stability.

However, technical problems to be solved in the present invention are not limited to the above-described problems, and other problems which are not described herein will be fully understood by those of ordinary skill in the art from the following descriptions.

### [Technical Solution]

To attain the above-described purposes,
the present invention provides a liquid composition for stabilizing botulinum toxin, which includes polysorbate 80 and serine as active ingredients.

In one embodiment of the present invention, in the composition, the composition ratio of polysorbate 80 and serine is 1 (mg/mL): 5 (mM) or more.

In another embodiment of the present invention, the composition further includes methionine.

In still another embodiment of the present invention, in the composition, the composition ratio of polysorbate 80, serine and methionine is 1 (mg/mL): 5 to 50 (mM) : 0.2 (mM).

In addition, the present invention provides a pharmaceutical composition in liquid formulation, which includes a liquid composition for stabilizing botulinum toxin; and botulinum toxin as active ingredients.

In one embodiment of the present invention, the concentration of polysorbate 80 is 0.001 mg or more per 40 units of botulinum toxin.

In one embodiment of the present invention, the concentration of serine is 5 mM or more per 40 units of botulinum toxin.

In one embodiment of the present invention, the concentration of methionine is less than 0.5 mM per 40 units of botulinum toxin.

In one embodiment of the present invention, the botulinum toxin is selected from the group consisting of botulinum type A, B, C, D, E, F, and G.

Preferably, the botulinum toxin is in a form that does not contain a complexing protein or a complex form that contains a complexing protein.

In addition, the present invention provides a method of stabilizing botulinum toxin, which includes mixing a liquid composition containing polysorbate 80 and serine with botulinum toxin.

In addition, the present invention provides a use of a liquid composition containing polysorbate 80 and serine for stabilizing botulinum toxin.

In addition, the present invention provides a use of polysorbate 80 and serine for producing a drug used to stabilize botulinum toxin.

### [Advantageous Effects]

The present invention uses a stabilizer which is not derived from an animal source, and thus a patient administered a botulinum toxin pharmaceutical composition can secure safety from the potential risk of infection with a blood-derived pathogen or infectious microorganism. In addition, the present invention can ensure storage and distribution stability since the activity of botulinum toxin is maintained for a long time at room temperature. In addition, the present invention has improved convenience in preparation since there is no need for a freeze-drying process.

### [Description of Drawings]

FIG. 1 is a graph showing the change in mortality rate when a botulinum toxin solution is administered according to the change in concentration of polysorbate 80.
FIG. 2 is a graph showing the change in mortality rate when a botulinum toxin solution is administered according to the change in concentration of serine.
FIG. 3 is a graph showing the change in mortality rate when a botulinum toxin solution is administered according to the change in concentration of serine due to the addition of methionine.

### [Modes of the Invention]

Hereinafter, to help in understanding the present invention, exemplary examples will be suggested. However, the following examples are merely provided to more easily understand the present invention, and not to limit the present invention.

The present invention relates to a liquid composition for stabilizing botulinum toxin, which includes polysorbate 80 and serine as active ingredients; and a pharmaceutical composition in liquid formulation, which includes the liquid composition for stabilizing botulinum toxin and botulinum toxin as active ingredients.

In addition, the present invention relates to a method of improving the stability of botulinum toxin using polysorbate 80 and serine.

The inventors conducted an experiment for ensuring a stabilizer which is safe since an animal-derived protein is not used, and has long-term stability at room temperature or even in storage in a refrigerator, as an alternative to a liquid composition for stabilizing botulinum toxin consisting of the combination of methionine and polysorbate 20. As a result, it was confirmed that the combination of serine and polysorbate 80 has an excellent effect of maintaining the activity of botulinum toxin at room temperature for a long time, and the present invention was completed.

In the present invention, the "stability or stabilization" means that the activity of botulinum toxin is maintained, and for example, the activity of botulinum toxin is maintained to be 10% or more, 20% or more, 30% or more, 40% or more, 50% or more, 60% or more, 70% or more, 80% or more, 90% or more, or 100%, preferably, 50% or more, and most preferably, 100%, compared with that before the experiment or under specific conditions. For example, compared with the number of subjects into which botulinum toxin is injected, a value obtained by (the number of dead subjects among subjects into which botulinum toxin is injected/the number of subjects into which botulinum toxin is injected) x 100 is maintained to be 10% or more, 20% or more, 30% or more, 40% or more, 50% or more, 60% or more, 70% or more, 80% or more, 90% or more, or 100%, preferably, 50% or more, and most preferably, 100%. The activity of botulinum toxin may be measured according to various methods known in the art, and for example, measured with reference to the lethality assay method described in PearceL.B.et al 1994. Measurement of Botulinum Toxin Activity: Evaluation of the Lethality Assay. Toxicology and Applied Pharmacology' 128: 69-77'. According to an experimental example of the present invention, 0.1 mL (3 or 4 units/0.1 mL/mouse) of the prepared botulinum toxin liquid composition was intraperitoneally injected into five or ten 4-week-old ICR (Institute of Cancer Research, USA) female mice, and then observed for 72 hours to confirm a mortality rate. In addition, the condition for "stability or stabilization" may refer to storage or distribution stability at room temperature, but the present invention is not limited thereto. According to a specific embodiment, the stability or stabilization condition may be a 35 to 45 °C/50 to 80% RH condition, and preferably, a 40 °C/70% RH condition.

The liquid composition for stabilizing botulinum toxin according to the present invention includes polysorbate 80 and serine as active ingredients.

In addition, the pharmaceutical composition in liquid formulation according to the present invention includes the liquid composition for stabilizing botulinum toxin and botulinum toxin as active ingredients. That is, the pharmaceutical composition in liquid formulation includes polysorbate 80 and serine; and botulinum toxin as active ingredients.

In the present invention, the "serine" is a compound represented by the chemical formula HO₂CCH(NH₂)CH₂OH, and includes a stereoisomer thereof. The "serine" may be obtained by various methods known in the art, which include a chemical synthesis method, a method of separating serine synthesized in an animal body, or a method of obtaining a commercially available compound, and for example, serine may be prepared by a method of removing 3-phosphoserine and a phosphoric acid group by an amino group transfer reaction with glutamic acid starting from oxidation of 3-phosphoglycerate formed from 3-phosphohydroxypyruvate and nicotinamide adenine dinucleotide (NADH), but the present invention is not limited thereto. In the present invention, a serine concentration included in the pharmaceutical composition in liquid formulation may be 5 mM or more per 40 units of botulinum toxin, preferably, 5 to 100 mM per 40 units of botulinum toxin, more preferably, 15 to 100 mM per 40 units of botulinum toxin, and most preferably, 15 to 50 mM per 40 units of botulinum toxin. At the concentration of less than 5 mM per 40 units of botulinum toxin, the stabilization of botulinum toxin caused by serine may be exhibited at lower than a predetermined level in long-term storage, and at the concentration of more than 100 mM per 40 units of botulinum toxin, a synergistic effect caused by excessive serine may be no longer exhibited, and may not be economical.

In the present invention, the "polysorbate 80" is a compound represented by the chemical formula C₆₄H₁₂₄O₂₆, is a non-ionic surfactant, and is soluble. The polysorbate 80 is called monooleic acid polyoxyethylene sorbitan, polyoxyethylene sorbitan monooleate, emazole 4130, Tween 80, or Tween 79, and used as a food or cosmetic additive for the purpose of, for example, a plasticizer, a gelling agent, a surfactant, a base, a sugar-coated preparation, a water-proofing agent, an anti-adhesive agent, an excipient, a dispersing agent, a disintegrant, a wetting agent, a stabilizer, a softening agent, a buffer, a solvent, a solubilizing agent, an emulsifying agent, an enteric coating agent, a coating agent, a bleaching agent, a suspending agent or a lubricant. The polysorbate 80 may be prepared by various methods known in the art, and may be isolated in nature or chemically synthesized. In the present invention, the concentration of polysorbate 80 included in the pharmaceutical composition in liquid formulation may be 0.001 mg or more per 40 units of botulinum toxin, preferably, 0.1 to 5 mg per 40 units of botulinum toxin, more preferably 0.1 to 2 mg per 40 units of botulinum toxin, and most preferably, 0.5 to 2 mg per 40 units of botulinum toxin. At less than 0.001 mg per 40 units of botulinum toxin, the stabilization of botulinum toxin by polysorbate 80 may be exhibited at less than a predetermined level in long-term storage, and at more than 5 mg per 40 units of botulinum toxin, a synergistic effect caused by excessive polysorbate 80 may be no longer exhibited, and may not be economical.

In the liquid composition for stabilizing botulinum toxin of the present invention, the composition ratio of polysorbate 80: serine per 20 units of botulinum toxin may be 1 (mg/mL): 5 (mM) or more, preferably, 1 (mg/mL): 5 to 50 (mM), and more preferably, 1 (mg/mL): 15 to 50 (mM). When the composition ratio of polysorbate 80 : serine is 1 (mg/mL): less than 5 (mM), the stabilization of botulinum toxin may be exhibited at less than a predetermined level in long-term storage.

In the present invention, the "botulinum toxin" may be randomly selected from the group consisting of botulinum type A, B, C, D, E, F and G, and preferably botulinum type A. The botulinum toxin may include both a form that does not contain a complexing protein and a complex form that contains a complexing protein. The molecular weight of a botulinum toxin protein derived from *Clostridium botulinum* type A, B, C, D, E, F or G, which does not contain a naturally-occurring complexing protein, is approximately 150 KDa. However, when a toxin protein is produced by *Clostridium botulinum,* a botulinum toxin protein is produced by forming various complexes with several hemagglutinin proteins that assist and protect the action of the botulinum toxin protein and non-hemagglutinin proteins. Botulinum toxin type A containing a naturally-occurring complexing protein is a complex having a molecular weight of approximately 900 kDa, 500 kDa or 300 kDa, botulinum toxin types B and C are complexes having a molecular weight of approximately 500 kDa, botulinum toxin type D is a complex having a molecular weight of approximately 300 kDa or 500 kDa, and botulinum toxin types E and F are complexes having a molecular weight of approximately 300 kDa. In the present invention, 1 unit of botulinum toxin may be defined as LD₅₀, in which a mortality rate caused by intraperitoneal injection into female Swiss Webster mice weighing 18 to 20 g is 50%.

As an active ingredient for the liquid composition for stabilizing botulinum toxin according to the present invention, methionine may be further included in addition to serine and polysorbate 80.

In the present invention, the concentration of methionine included in the pharmaceutical composition in liquid formulation may be less than 0.5 mM per 40 units of botulinum toxin, and more preferably, 0.2 to 0.4 mM per 40 units of botulinum toxin, but the present invention is not limited thereto.

In the liquid composition for stabilizing botulinum toxin of the present invention, the composition ratio of polysorbate 80 : serine : methionine per 20 units of botulinum toxin may be 1 (mg/mL): 2.5 to 50 (mM) : 0.2 (mM), preferably 1 (mg/mL): 5 to 50 (mM) : 0.2 (mM), more preferably 1 (mg/mL): 7.5 to 30 (mM) : 0.2 (mM), and further more preferably 1 (mg/mL): 7.5 to 25 (mM) : 0.2 (mM). When the composition ratio of polysorbate 80 : serine : methionine is 1 (mg/mL): less than 2.5 or more than 50 (mM) : 0.2 (mM), the stabilization of botulinum toxin may be exhibited at less than a predetermined level in long-term storage.

According to the present invention, the liquid composition for stabilizing botulinum toxin and/or the pharmaceutical composition in liquid formulation including the same may include a pharmaceutically acceptable carrier in addition to the active ingredients. Here, the pharmaceutically acceptable carrier is conventionally used in preparation, and includes, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propyl hydroxybenzoate, talc, magnesium stearate, and mineral oil, but the present invention is not limited thereto. In addition, the liquid composition for stabilizing botulinum toxin and/or pharmaceutical composition in liquid formulation of the present invention may further include a lubricant, a wetting agent, a sweetening agent, a flavoring agent, an emulsifier, a suspending agent or a preservative, in addition to the above-described ingredients.

The pharmaceutical composition of the present invention may be orally or parenterally (e.g., intravenously, subcutaneously, intraperitoneally or locally) administered by a desired method, and a dose may be suitably selected by those of ordinary skill in the art according to the condition and body weight of a patient, the severity of a disease, a dosage form, the route and duration of administration.

The composition according to the present invention is administered at a pharmaceutically effective amount. In the present invention, the "pharmaceutically effective amount" used herein refers to an amount sufficient for treating a disease at a reasonable benefit/risk ratio applicable for medical treatment, and an effective dosage may be determined by parameters including the type of a patient's disease, severity, drug activity, sensitivity to a drug, administration time, an administration route and an excretion rate, the duration of treatment and drugs simultaneously used, and other parameters well known in the medical field.

The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without a side effect, and such a dose may be easily determined by one of ordinary skill in the art. The co-administered preparation is not limited, and for example, the second composition may be an aminoglycoside-based antibiotic or a drug that interferences with muscle and neurotransmission (tubocuraline-based muscle relaxant).

In addition, the present invention provides a method of stabilizing botulinum toxin, which includes mixing a liquid composition including polysorbate 80 and serine with botulinum toxin.

In addition, the present invention provides a use of a liquid composition including polysorbate 80 and serine for stabilizing botulinum toxin and a use of polysorbate 80 and serine for producing a drug used to stabilize botulinum toxin.

In addition, the "subject" used herein refers to a target in need of administration of botulinum toxin, and more specifically, and includes a mammal such as a human or a non-human primate, a mouse, a rat, a dog, a cat, a horse, or a cow.

The "administration" used herein refers to the provision of the liquid composition including polysorbate 80 and serine; and botulinum toxin to a subject by a suitable method.

The liquid composition for stabilizing botulinum toxin according to the present invention is prepared by mixing serine and polysorbate 80.

In addition, the pharmaceutical composition in liquid formulation according to the present invention is prepared by preparing a liquid composition for stabilizing botulinum toxin by mixing serine and polysorbate 80; and mixing the liquid composition for stabilizing botulinum toxin and botulinum toxin.

As needed, in addition to the above-described steps, one or more steps may be further included, and the sequence is irrelevant. For example, the liquid composition for stabilizing botulinum toxin may include additionally mixing methionine in the step of mixing serine and polysorbate 80.

### <Example>

### 1. Preparation of liquid composition containing botulinum toxin

A liquid composition containing botulinum toxin was prepared by diluting a botulinum toxin solution using a liquid composition for stabilizing botulinum toxin adjusted to a proper concentration and finally adjusting the concentration of the botulinum toxin solution to a proper concentration.

### <Experimental Examples>

### 1. Test for selecting liquid composition for stabilizing botulinum toxin

To select a stabilizer of *Clostridium botulinum* toxin type A, an experiment for selecting the composition of a liquid composition for stabilizing botulinum toxin was performed using various stabilizers.

Using a liquid composition for stabilizing botulinum toxin having the composition as shown in Table 1 below, a botulinum toxin solution was prepared to have a concentration of 300 Unit/mL.

Like the patented Innotox formulation, a control was prepared by dissolving 20 mM methionine and 2 mg/mL polysorbate 20 in 10% saline. In addition, experimental groups (Examples 1 to 7) were prepared by dissolving each a 20 mM stabilizer in normal saline and dissolving 2 mg/mL of polysorbate 80 in 10% saline.

The prepared botulinum toxin solution was stored in a stability chamber under conditions including 40 °C/70% RH, and sampled at regular intervals. 9 mL of an injectable solution (normal saline) was added to 1 mL of the sampled botulinum toxin solution to dilute the solution 10-fold, and the diluted sample was intraperitoneally injected into five 4-week-old ICR (Institute of Cancer Research, USA) female mice at 3 units/0.1 mL (1 unit = LD₅₀). While observing for 72 hours after the intraperitoneal injection, the number of dead mice and the mortality rate corresponding thereto were assessed.

As a result, as shown in Table 1, other than Examples 1 and 4, and Control, a dramatic decrease in stability was confirmed. That is, the activity of botulinum toxin was maintained for a long time only in Examples 1 and 4 and Control.

From the above result, it can be seen that the combination of serine and polysorbate 80 is a stabilizer that can replace the combination of methionine and polysorbate 80.

**[Table 1]**

| **Classification** | **Liquid composition for stabilizing botulinum toxin** | | **Mortality rate (%)** | | | **Stability** |
|---|---|---|---|---|---|---|
| | **Stabilizer (20 mM)** | **Polysorbate (2 mg/mL)** | **0 week** | **1^{st} week** | **2^{nd} week** | |
| Control | Methionine | Tween 20 | 100% | 100% | 100% | ○ |
| Example 1 | Methionine | Tween 80 | 100% | 100% | 100% | ○ |
| Example 2 | Glycine | Tween 80 | 100% | 100% | 0% | X |
| Example 3 | Cysteine | Tween 80 | 100% | 0% | 0% | X |
| Example 4 | Serine | Tween 80 | 100% | 100% | 100% | ○ |
| Example 5 | Zn (50 mM) | Tween 80 | 100% | 40% | 0% | X |
| Example 6 | Leucine | Tween 80 | 80% | 60% | 0% | X |
| Example 7 | Vitamin C | Tween 80 | 80% | 0% | 0% | X |

However, the stability experiment was performed on live mice, and due to its characteristic, even when the stability experiment was conducted with a botulinum toxin liquid composition having the same composition, different results may be obtained depending on the condition of a mouse.

### 2. Test for stability of botulinum toxin according to change in concentration of polysorbate 80

As the composition of the liquid composition for stabilizing botulinum toxin, the combination of serine and polysorbate 80 was selected, and an experiment for the stability of botulinum toxin according to the change in concentration of polysorbate 80 was performed.

Using a liquid composition for stabilizing botulinum toxin having the composition as described in Table 2 below, a botulinum toxin solution was prepared to have a concentration of 40 units/mL. An experimental group was prepared by dissolving 5 mM serine and 0.001 to 2 mg/mL of polysorbate 80 in 10% normal saline. A specific experimental method was carried out as described in Experimental Example 1. However, this method used 10 mice, and a concentration for intraperitoneal injection was 4 units/0.1 mL.

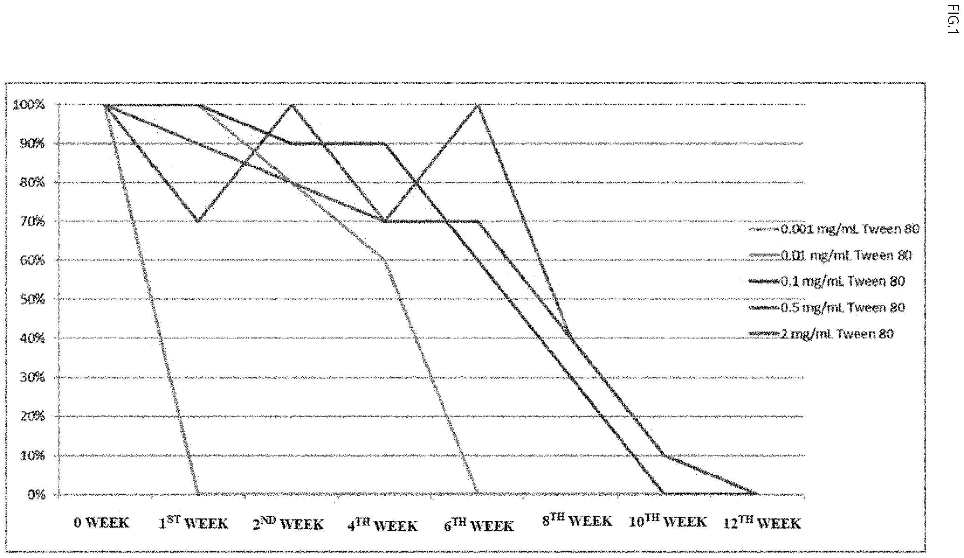

The result of an experiment for the stability of botulinum toxin according to the change in concentration of polysorbate 80 is shown in Table 2 and FIG. 1. When the concentration of polysorbate 80 is 0.1 mg/mL or more, a certain degree or more of stability was shown at every concentration. When the concentration of polysorbate 80 is 0.1 mg/mL, 0.5 mg/mL and 2 mg/mL, a mortality rate was 50% or more until the 6^{th} week, and when the concentration of polysorbate 80 was 0.5 mg/mL or 2 mg/mL, a mortality rate was 10% until the 10^{th} week.

**[Table 2]**

| **Botulinum toxin solution** | | | **Mortality rate (%)** | | | | | | | |
|---|---|---|---|---|---|---|---|---|---|---|
| **Liquid composition for stabilizing botulinum toxin** | | **Toxin (U/mL)** | **0 week** | **1^{st} week** | **2^{nd} week** | **4^{th} week** | **6^{th} week** | **8^{th} week** | **10^{th} week** | **12^{th} week** |
| **Polysorbate 80 (mg/mL)** | **serine (mM)** | | | | | | | | | |
| 0.001 | 5 | 40 | 100% | 0% | 0% | 0% | 0% | 0% | 0% | 0% |
| 0.01 | | | 100% | 100% | 80% | 60% | 0% | 0% | 0% | 0% |
| 0.1 | | | 100% | 100% | 90% | 90% | 60% | 30% | 0% | 0% |
| 0.5 | | | 100% | 90% | 80% | 70% | 100% | 40% | 10% | 0% |
| 2 | | | 100% | 70% | 100% | 70% | 70% | 40% | 10% | 0% |

### 3. Test for stability of botulinum toxin according to change in concentration of serine

An experiment for the stability of botulinum toxin according to the change in concentration of serine was performed with the combination of serine and polysorbate 80 selected as the composition of a liquid composition for stabilizing botulinum toxin.

Using a liquid composition for stabilizing botulinum toxin having the composition as described in Table 3 below, a botulinum toxin solution was prepared to have a concentration of 40 Unit/mL. A control was prepared by dissolving 20 mM methionine and 2 mg/mL of polysorbate 20 in 10% saline like the patented Innotox formulation. An experimental group was prepared by dissolving 0.1 mg/mL of polysorbate 80 and 5 to 50 mM serine in 10% normal saline. A specific experimental method was carried out as described in Experimental Example 1. However, this method used 10 mice, and a concentration for intraperitoneal injection was 4 units/0.1 mL.

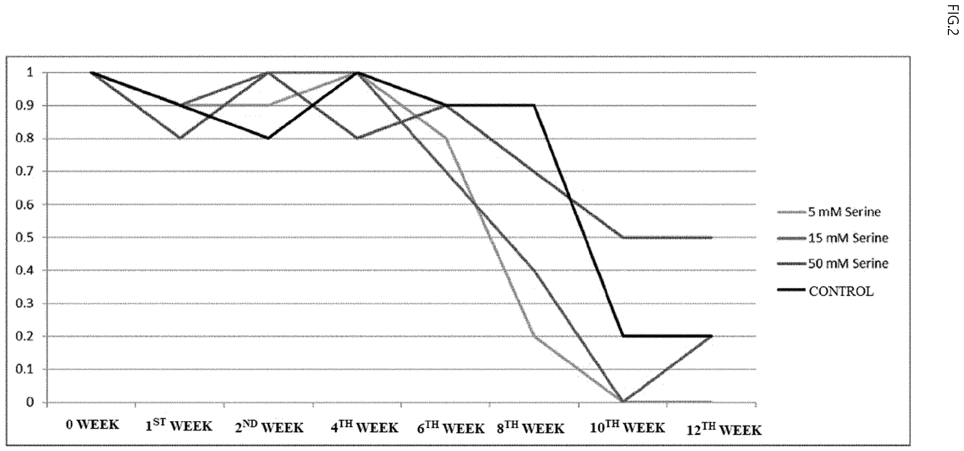

The result of an experiment for the stability of botulinum toxin according to the change in concentration of serine is shown in Table 3 and FIG. 2. When the concentration of serine is 5 mM, 15 mM or 50 mM, the mortality rate was 50% or more until the 6^{th} week, showing that the activity of botulinum toxin is maintained. Particularly, when the concentration of serine is 50 mM, the mortality rate was 50% or more until the 12^{th} week, showing that the stability of botulinum toxin is maintained for a long time compared with the combination of 20 mM methionine and 2 mg/ml of polysorbate 20.

**[Table 3]**

| **Liquid composition for stabilizing botulinum toxin** | | **Mortality rate (%)** | | | | | | | |
|---|---|---|---|---|---|---|---|---|---|
| **Serine (mM)** | **Polysorbate 80 (mg/mL)** | **0 week** | **1^{st} week** | **2^{nd} week** | **4^{th} week** | **6^{th} week** | **8^{th} week** | **10^{th} week** | **12^{th} week** |
| 5 | 0.1 | 100% | 90% | 90% | 100% | 80% | 20% | 0% | 0% |
| 15 | | 100% | 90% | 100% | 100% | 70% | 40% | 0% | 20% |
| 50 | | 100% | 80% | 100% | 80% | 90% | 70% | 50% | 50% |
| 20mM methionine,2mg/mL polysorbate20 | | 100% | 90% | 80% | 100% | 90% | 90% | 20% | 20% |

### 4. Test for stability of botulinum toxin according to addition of methionine

An experiment for the stability of botulinum toxin according to the addition of methionine was performed with the combination of serine and polysorbate 80 selected as the composition of a liquid composition for stabilizing botulinum toxin.

Using a liquid composition for stabilizing botulinum toxin having the composition as described in Table 4 below, a botulinum toxin solution was prepared to have a concentration of 40 units/mL. A control was prepared by dissolving 20 mM methionine and 2 mg/mL of polysorbate 20 in 10% saline like the patented Innotox formulation. An experimental group was prepared by dissolving 2 mg/mL of polysorbate 80, 5 to 50 mM serine and 0.4 mM methionine in 10% normal saline. A specific experimental method was carried out as described in Experimental Example 1. However, this method used 10 mice, and a concentration for intraperitoneal injection was 4 units/0.1 mL.

**[Table 4]**

| Botulinum toxin solution | | | | Mortality rate (%) | | | | | | | Stability |
|---|---|---|---|---|---|---|---|---|---|---|---|
| Liquid composition for stabilizing botulinum toxin | | | Toxin (U/mL) | 0 week | 2^{nd} week | 4^{th} week | 6^{th} week | 8^{th} week | 10^{th} week | 12^{th} week | |
| Methionine (mM) | Serine (mM) | Polysorbate 80 (mg/mL) | | | | | | | | | |
| 0.4 | 5 | 2 | 40 | 100 | 100 | 70 | 70 | 80 | 70 | 70 | △ |
| 0.4 | 15 | 2 | | 100 | 100 | 80 | 90 | 80 | 80 | 70 | △ |
| 0.4 | 50 | 2 | | 100 | 100 | 80 | 80 | 90 | 80 | 80 | ○ |
| Methionine 20mM, Polysorbate 20 2mg/mL | | | | 100 | 100 | 80 | 90 | 80 | 60 | 60 | △ |

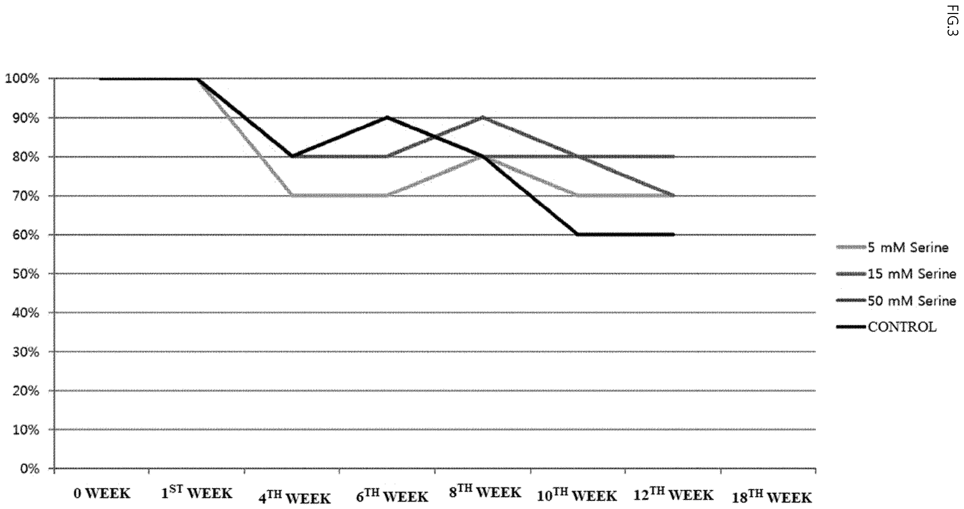

As a result, as shown in Table 4 and FIG. 3, it can be seen that the stability of the botulinum toxin solution was improved by the combination of methionine, serine and polysorbate 80 in all formulations, compared with a control.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

Polysorbate 80 and serine according to the present invention are expected to be effectively used in a composition for improving the stability and ease of administration of liquid botulinum toxin.

## Claims

1. A liquid composition for stabilizing botulinum toxin, comprising polysorbate 80 and serine as active ingredients.

2. The composition of claim 1, wherein the composition ratio of polysorbate 80 : serine is 1 (mg/mL): 5 (mM) or more.

3. The composition of claim 1, further comprising methionine.

4. The composition of claim 3, wherein the composition ratio of polysorbate 80 : serine: methionine is 1 (mg/mL): 5 to 50 (mM) : 0.2 (mM).

5. A pharmaceutical composition in liquid formulation, comprising the liquid composition for stabilizing botulinum toxin of any one of claims 1 to 4; and botulinum toxin as active ingredients.

6. The pharmaceutical composition of claim 5, wherein the concentration of polysorbate 80 is 0.001 mg or more per 40 units of the botulinum toxin.

7. The pharmaceutical composition of claim 5, wherein the concentration of serine is 5 mM or more per 40 units of the botulinum toxin.

8. The pharmaceutical composition of claim 5, wherein the concentration of methionine is less than 0.5 mM per 40 units of the botulinum toxin.

9. The pharmaceutical composition of claim 5, wherein the botulinum toxin is selected from the group consisting of botulinum type A, B, C, D, E, F and G.

10. The pharmaceutical composition of claim 5, wherein the botulinum toxin is a form that does not contain a complexing protein or a complex form that contains a complexing protein.

11. A method for stabilizing botulinum toxin, comprising:
mixing a liquid composition comprising polysorbate 80 and serine with botulinum toxin.

12. A use of a liquid composition comprising polysorbate 80 and serine for stabilizing botulinum toxin.
